# EUROPÄISCHE PATENTSCHRIFT

(11) **EP 3 032 949 B1**
(45) Veröffentlichungstag und Bekanntmachung des Hinweises auf die Patenterteilung: **03.03.2021**
(21) Anmeldenummer: 14755619.5
(22) Anmeldetag: 13.08.2014
(51) Int. Cl.: A01N 31/08, A61L 31/16, A61L 27/54, A01N 25/28, A01N 25/10, A01P 1/00, C09D 5/14

(54) **MITTEL ENTHALTEND MIKROPARTIKEL ZUR REINIGUNG UND ZUM SCHUTZ VON TECHNISCHEN OBERFLÄCHEN**
COMPOSITION COMPRISING MICROPARTICLES FOR CLEANING AND PROTECTION OF TECHNICAL MATERIAL
AGENT CONTENANT DES MICROPARTICULES, SERVANT À NETTOYER ET À PROTÉGER DES SURFACES TECHNIQUES

(30) Priorität: 16.08.2013 DE 102013108870
(43) Veröffentlichungstag der Anmeldung: 22.06.2016
(73) Patentinhaber: Aimecs GmbH, 84347 Pfarrkirchen (DE)
(72) Erfinder: WENDEL, Hans-Peter, 72336 Balingen (DE); WEINDL, Juergen, 84347 Pfarrkirchen (DE)
(74) Vertreter: Witte, Weller & Partner Patentanwälte mbB
(86) Internationale Anmeldenummer: PCT/EP2014/067335
(87) Internationale Veröffentlichungsnummer: WO 2015/022366

(56) Entgegenhaltungen:
- WO-A1-2013/089721
- WO-A2-2006/002365
- US-A1- 2009 047 316
- US-A1- 2010 003 300
- US-A1- 2011 206 773

## Beschreibung

Die vorliegende Erfindung betrifft ein Mittel zur reinigenden, desinfizierenden und/oder schützenden Behandlung von technischen Oberflächen, mit zumindest einem antibakteriell wirkenden Inhaltsstoff.

Derartige Mittel sind aus dem Alltag vielfältig bekannt. Sie enthalten antibakteriell wirkende Inhaltsstoffe wie Alkohole, Tenside, etc.

Die bekannten Mittel werden verwendet, um in Industrie, Haushalt und Krankenhäusern die technischen Oberflächen nach dem ersten Reinigen, also dem Entfernen von Schmutzpartikeln und sonstigen Verunreinigungen, auch von Mikroorganismen und Krankheitskeimen zu befreien und sie ggf. für eine gewisse Zeit vor erneutem Befall mit Mikroorganismen und sonstigen Keimen zu schützen.

Im Rahmen der vorliegenden Erfindung wird unter einer "technischen Oberfläche" eine nicht biologische Oberfläche verstanden, die einer regelmäßigen oder einmaligen Desinfizierung bedarf. Zu diesen technischen Oberflächen zählen beispielsweise aber nicht abschließend die Oberflächen von Tischen, Türklinken, Stühlen, Ablagen, Betten, technischen Geräten, Haushaltsgeräten, Telefonen, Fenstern, Toiletten, Bürogeräten, Bädern, Küchen, Operationssälen, Operationsgeräten, und Operationshandschuhen.

Die Firma Neroform AG bietet zum Beispiel für die Reinigung von Oberflächen in Büro, Krankenhaus und Haushalt das Desinfektionsmittel *Neroform* G an, das innerhalb von 60 Sekunden 98% aller Bakterien vernichten und durch Langzeitwirkung die Neubildung von Bakterienherden während über einem Monat fast gänzlich verhindern soll. *Neroform* G soll vor ansteckenden Krankheiten, Bakterien und Pilzen schützen.

*Neroform* G ist als Desinfektionsspray, getränkte Tücher und flüssiges Desinfektionsmittel verfügbar. Der Inhaltsstoff von *Neroform* G und dessen Formulierung sind der Anmelderin nicht bekannt.

Die Effizienz derartiger Mittel und deren Anwendungsbereiche sowie Langzeitwirkungen lassen häufig zu wünschen übrig. Ferner entwickeln viele Mikroorganismen im Laufe der Zeit gewisse Resistenzen gegenüber den eingesetzten Desinfektionsmitteln, so dass ein fortlaufender Bedarf an neuen und verbesserten Mitteln der eingangs genannten Art besteht.

Der vorliegenden Erfindung liegt daher die Aufgabe zugrunde, ein neues Mittel der eingangs genannten Art zu schaffen.

Diese Aufgabe wird erfindungsgemäß gelöst durch ein Mittel der eingangs genannten Art, das mit dem antibakteriell wirkenden Inhaltsstoff beladene Mikropartikel enthält, wobei die Mikropartikel aus einem Poly (Lactid-co-glycolid) mit einem Lactid-Glycolid-Verhältnis von 50:50 und einem Molekulargewicht kleiner als 30.000g/Mol hergestellt sind, und dass der Inhaltsstoff zumindest Totarol umfasst.

Unter "Mikropartikeln" werden erfindungsgemäß meist kugelförmige Feststoffpartikel in einem Größenbereich zwischen 1 und 1000 µm verstanden. Sie basieren in der Regel auf bioabbaubaren, biokompatiblen Polymeren wie PLA (Polymilchsäure = Poly (lactic acid)) oder PLGA (Copolymerisate aus Milch- und Glycolsäure =

Poly(lactic-co-glycolic acid)). Derartige Mikropartikel zählen zu den parenteralen Depotarzneiformen, die sich bereits am Markt etabliert haben.

Aus der US 2010/0003300 A1 ist es bekannt, derartige Mikropartikel mit therapeutischen, prophylaktischen oder diagnostischen, bioaktiven Reagenzien, beispielsweise mit antibiotischen oder antimikrobiellen Agenzien, zu beladen und die so hergestellte Zusammensetzung einem Patienten über Nadeln mit geringem Durchmesser zu injizieren. Die Zusammensetzung kann dabei pharmazeutisch akzeptable Träger, Additive und/oder Lösungsmittel enthalten.
Mikropartikel, die Poly (DL-Lactid-co-glycolid) als Polymer enthalten bzw. daraus hergestellt sind, werden offenbart in den US 2010/0003300 WO 2006/002365 und US 2011/206773.

Die Zusammensetzung soll eine kontrollierte und ggf. verzögerte Freisetzung der Reagenzien im Körper des Patienten bewirken.

Die Zusammensetzung ist auch als Beschichtung für medizinische Geräte und Implantate, beispielsweise für Herzklappen und Gefäßprothesen vorgesehen.

Mikropartikel zeichnen sich durch eine Reihe von Vorteilen aus, wie z. B. einer verbesserten Bioverfügbarkeit, einer Reduzierung systemischer Nebenwirkungen sowie einer Verbesserung der Therapietreue und des Komforts der Patienten.

Immer mehr wissenschaftliche Gruppen zeigen verstärktes Interesse für den Einschluss von hydrophoben bzw. hydrophilen Wirkstoffen sowie Biologicals zur langfristigen Behandlung chronischer und neurodegenerativer Erkrankungen, wie z. B. Diabetes mellitus und Morbus Parkinson.

Mikropartikel werden mit unterschiedlichsten Verfahren hergestellt. Meist ist das Verfahren an den jeweiligen Wirkstoff und seine physikalisch-chemischen Eigenschaften angepasst. Das Herstellverfahren entscheidet, welche der zwei Arten von Mikropartikeln dabei entsteht. Es wird unterschieden zwischen Mikrokapseln, bestehend aus Kern- und Mantelmaterial, und Mikrosphären/-sphärulen (microspheres), bestehend aus einer Polymermatrix.

Das Wandmaterial der Mikrokapseln umschließt das feste, flüssige bzw. gasförmige Kernmaterial, in dem der Wirkstoff enthalten ist. Zur Herstellung von Mikrokapseln werden im pharmazeutischen Bereich hauptsächlich das Koazervationsverfahren, das Emulsionsverfahren mit Lösungsmittelverdampfung/Extraktion und die Sprühtrocknung eingesetzt.

Als Mikrosphärulen werden Mikropartikel mit einem Durchmesser kleiner als etwa 250 µm bezeichnet, in deren Polymermatrix Wirkstoffe möglichst homogen eingebettet sind. Sie sind für eine Wirkstofffreisetzung über mehrere Wochen besonders geeignet. Mikrosphärulen entstehen bei einem Emulsionsverfahren, bei dem das Lösungsmittel, in dem das Polymer gelöst ist, relativ schnell verdampft oder extrahiert wird.

Zur Herstellung des erfindungsgemäßen Mittels stehen zahlreiche Emulsionsverfahren zur Verfügung. Die klassischen Emulsionen sind die Öl-in-Wasser-Emulsionen (O/W-Emulsion) und die Wasser-in-ÖI-Emulsionen (W/O-Emulsion). Die zuerst genannte Emulsion eignet sich besonders gut, um hydrophobe Wirkstoffe in Mikropartikeln einzuschließen, dabei werden Einschlusseffizienzen von mehr als 90 % erreicht, indem der Wirkstoff in der organischen Phase gelöst oder dispergiert wird.

Mit der W/O-Emulsion sind solche Ergebnisse weder für hydrophile noch für hydrophobe Wirkstoffe erreichbar. Da sich hydrophile Wirkstoffe in der organischen Phase nicht bzw. sehr schlecht lösen, wird die einfache W/O-Emulsion um eine äußere Phase erweitert. So entstehen zwei weit verbreitete Arten multipler Emulsionen; zum einen die Wasser-in-ÖI-in-Wasser-Emulsion (kurz: W/O/W) und zum anderen die Wasser-in-Öl-in-Öl-Emulsion (kurz: W/O/O).

Die Erfinder haben erkannt, dass wider Erwarten für das eingangs genannte, außerhalb des Körpers für Reinigung, Desinfektion und Schutz von technischen Oberflächen vorgesehene Mittel derartige Mikropartikel als Träger für die Inhaltsstoffe verwendet werden können.

Unter "Reinigung, Desinfektion und Schutz" wird im Rahmen der vorliegenden Erfindung auch die Dekontamination von technischen Oberflächen oder Geräten verstanden.

Durch das Beladen der Mikropartikel mit den Inhaltsstoffen erfolgt auch außerhalb des Körpers eine kontinuierliche Freisetzung des Wirkstoffes über Wochen, so dass auch nach mehreren Wochen noch eine hinreichende Desinfektionswirkung gewährleistet ist.

Die Freisetzung der Inhaltsstoffe erfolgt also nicht zu Beginn der Anwendung schlagartig und dann mit abnehmender Geschwindigkeit, oder s-förmig, also am Anfang und am Ende der Wirkungsdauer mit großer Geschwindigkeit und dazwischen mit geringer Geschwindigkeit. Die Freisetzungskinetik ist vielmehr über dem Wirkungszeitraum gleichmäßig oder stetig, wobei es auch möglich ist, am Anfang der Wirkungsdauer eine größere Menge in einem sogenannten Burst freizusetzen, der dann in eine kontinuierliche Freisetzung übergeht.

Als Inhaltsstoff kommen dabei unterschiedliche antibakteriell wirkende Verbindungen und deren Mischungen in Frage. Erfindungsgemäss wird Totarol verwendet. Dessen Verwendung in der Desinfektion von Oberflächen ist aus WO 2013/089721 bekannt.

Die der Erfindung zugrundeliegende Aufgabe wird auf diese Weise vollkommen gelöst.

Die vorliegende Erfindung betrifft ferner die Verwendung des erfindungsgemäßen Mittels zur Reinigung, zur Desinfektion, zur Dekontamination und/oder zum Schutz von technischen Oberflächen.

Dabei ist es bevorzugt, wenn die Mikropartikel biodegradierbare Mikrokapseln oder Mikrosphärulen sind.

PLGA hat eine weit verbreitete Anwendung in der Medizin als selbstauflösendes Nahtmaterial oder in der Pharmazie als Depotarzneimittel (Wirkstoff-Implantat) gefunden. Dieses Polymer besteht aus definierten Anteilen von Polymilchsäure (Lactid) und Polyglykolsäure. Das Verhältnis dieser Säuren und das Molekulargewicht des gesamten Polymers bestimmen die späteren Eigenschaften des Implantats.

Dabei sorgt ein hoher Lactid-Anteil für eine langsamere Degradation des Implantats, gleichzeitig aber auch für eine sehr langsame und geringe Wirkstofffreisetzung, die es erschwert, die minimale effektive Dosis des Wirkstoffes zu erreichen. Ein höherer bzw. ausgeglichener Glykolsäure-Anteil hingegen sorgt für eine schnellere Degradation und Wirkstofffreisetzung, wodurch ein gewünschter Effekt erreicht werden kann. Es gibt viele verschiedene PLGA-Polymere, die sich stark in ihrem Molekulargewicht und dem Lactid-Glycolid-Verhältnis unterscheiden und somit auch in der Anwendung.

Für eine Freisetzung der Inhaltsstoffe innerhalb von 6 - 15 Wochen eignet sich nach Kenntnis der Erfinder besonders das Polymer mit einem Lactid-Glycolid-Verhältnis von 50:50 und einem Molekulargewicht kleiner als 30.000 g/Mol.

Als besonders geeignet für die Herstellung der erfindungsgemäß beladenen Mikropartikel hat sich Resomer ® RG 502 H erwiesen, das von der Firma Evonik vertrieben wird.

Nach der Herstellung liegen die Mikropartikel in trockenem Zustand vor.

Dabei ist es bevorzugt, wenn das Mittel ein Lösungsmittel für die mit dem Inhaltsstoff beladenen Mikropartikel enthält, wobei vorzugsweise das Lösungsmittel ausgewählt ist, aus der Gruppe, die Hexan, Aceton, Ethanol, Acetonitril, Chloroform, DSMO (Dimethyl Sulfoxid), Methanol, Ethylacetat und das Tensid Polyoxyethylen(4)laurylether umfasst.

Wenn das neue Mittel aus dem Aufbewahrungsgefäß auf die zu behandelnde Oberfläche aufgegeben ist, was durch Wischen mit einem getränkten Tuch, Sprühen oder direkten Flüssigkeitsauftrag erfolgen kann, bildet sich durch das Abtrocknen eine Art Film aus den beladenen Mikropartikeln, die nun nach und nach über hydrolytische Vorhänge die Inhaltsstoffe freisetzen, die dann ihre antimikrobielle Wirkung entfalten können.

Das neue Mittel kann auch in einem getränkten Tuch, insbesondere Reinigungstuch, Wundauflage, Abdecktuch, einer getränkten Mundschutzmaske oder getränkter Operationskleidung vorliegen.

Der Inhaltsstoff umfasst zumindest Totarol, vorzugsweise synthetisch hergestelltes oder aus einer natürlichen Quelle gewonnenes Totarol.

Totarol ist ein trizyklisches aromatisches Diterpen, das als (+)-Totarol antimikrobielle und antioxidative Eigenschaften aufweist. Es wird seit vielen Jahren zur Verwendung in kosmetischen und pharmazeutischen Produkten vorgeschlagen. Die Cosmetic, Toilettry, and Fragrance Association führt Totarol unter dem CFTA Aktenzeichen 7277.

Wegen seiner stark wirksamen antioxidativen und antibakteriellen Eigenschaften werden Totarol-Extrakte bereits als Zusatzstoffe in Zahncremes und Kosmetika eingesetzt.

Totarol kann als Naturstoff aus verschiedenen Pflanzenmaterialien, insbesondere aus dem Kernholz einer neuseeländischen Steineibenart (Podocarpus totara) gewonnen werden.

Der IUPAC Name von Totarol lautet: (4bS,8aS)-4b,8,8-Trimethyl-1-propan-2-yl-5,6,7,8a,9,10-hexahydrophenanthren-2-ol. Die CAS Nummer ist 511-15-9.

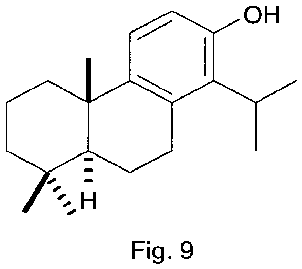

Totarol weist die in der Fig. 9 angegebene chemische Struktur auf.

Die WO 2005/073154 A1 beschreibt geeignete Verfahren zur Herstellung von Totarol enthaltenden Extrakten aus geeignetem Pflanzenmaterial. Diese Extrakte sollen zu lösungsmittelfreien Produkten weiterverarbeitet werden, die antimikrobiell gegen Grampositive und Gram-negative Bakterien wirken und als Reinigungs- und Desinfektionsmittel für Industrie und Haushalt, Kosmetika, Pharmazeutika, Haut- und Körperpflegemittel sowie für die Zahnpflege eingesetzt werden können.

Wenn sie als oral applizierbares Medikament formuliert sind, sollen die Produkte u.a. in Form von Kapseln, Tabletten, Pastillen, Sirup, Mundspülungen, Zahnpasten, Kaugummis und Mundsprays bereitgestellt werden.

Wenn sie als topisch applizierbare Formulierung verwendet werden, sollen die Produkte u.a. als Lotion, Creme, Gel, Spray, Reinigungsflüssigkeit, Shampoo, Puder, Hydrogel oder Wundauflage bereitgestellt werden.

Die WO 2005/073154 A1 beschreibt zwar geeignete Verfahren zur Herstellung der Extrakte, jedoch keine Verfahren zur Herstellung der Produkte.

Weil die natürlichen Resourcen von Totarol begrenzt sind, befasst sich die EP 2 143 703 B1 mit einem Verfahren zur chemischen Synthese von (+)-Totarol.

Aus der EP 1 925 301 A1 ist es bekannt, dass Totarol und dessen antimikrobiell wirkende Ester sowie deren Diastereomere zur Herstellung von pharmazeutischen Produkten und Nutraceuticals und zur Behandlung von entzündlichen Erkrankungen wie Arthritis verwendet werden können.

Die Synthese von antibakteriell wirksamen Totarol-Derivaten beschreiben Evans et. al. "The Synthesis and Antibacterial Activity of Totarol Derivatives", Part 1 in Bioorganic & Medicinal Chemistry 7 (1999), 1953 - 1964; Part 2 in Bioorganic & Medicinal Chemistry 8 (2000), 1653 - 1662; Part 3 in Bioorganic & Medicinal Chemistry 8 (2000), 1663 - 1975.

Totarol wirkt danach auch gegen multiresistente Staphylococcus aureaus (MRSA).

Die Autoren berichten über Versuche, die zeigen, dass die minimale Konzentration, in der Totarol *in vitro* noch antibakteriell wirkt, 2 ug/ml beträgt. Die Autoren zeigen ferner, dass Totarol in Konzentrationen oberhalb von 5 ug/ml toxisch und wachstumsinhibierend für humane Zellen wirkt. Sie schlagen weitere Experimente vor, um zu prüfen, ob der Bereich zwischen diesen Konzentrationen ein hinreichendes Sicherheitsfenster bietet, damit Totarol für die Behandlung von humanes bakteriellen Erkrankungen eingesetzt werden kann.

Die US 6,881,756 B2 beschreibt die Verwendung von Totarol und dessen pharmazeutisch wirksamen Estern zur topischen Behandlung von Schmerz und Juckreiz. Totarol wird dazu mit einem topischen Träger formuliert, für den als Beispiele Lösungen, Emulsionen, Gele, Mizellen und Liposomen genannt werden.

Die US 6,881,756 B2 beschreibt keine Verfahren zur Herstellung der Formulierungen.

Im Rahmen der vorliegenden Erfindung wird unter "Totarol" eine Verbindung der in Fig. 9 angegebenen Struktur verstanden.

Unter der erfindungsgemäß eingesetzten Totarol-Verbindung wird erfindungsgemäß ein Inhaltsstoff verstanden, der Totarol umfasst.

Die Erfinder haben erkannt, dass sich Totarol-Verbindungen auf einfache und effektive Weise auf Mikropartikel laden lassen, ohne dass die antibakterielle Wirkung beeinträchtigt wird.

Sie konnten in ersten Versuchen zeigen, dass mikroverkapseltes Totarol auch nach längerer Inkubationszeit mit Streptococcus gordonii noch antibakteriell wirkt. Die Wirkung des verkapselten Totarol war geringer als die von freiem Totarol, was an der langsamen Freisetzung von Totarol aus den Mikrokapseln liegt.

Wegen der oben beschriebenen antimikrobiellen Wirkung von Totarol-Verbindungen stellen die Erfinder damit ein neuartiges Mittel der eingangs genannten Art bereit, bei dem sich sowohl der Inhaltsstoff als auch dessen Bereitstellung in Form von beladenen Mikropartikeln vom Stand der Technik unterscheiden.

Die verzögerte Freisetzung der Totarol-Verbindung von den damit beladenen Mikropartikeln erlaubt nun zum einen die kontinuierliche Freisetzung von Totarol sowohl in vitro als auch *in vivo,* so dass über einen längeren Zeitraum die antibakterielle Wirkung aufrecht erhalten werden kann. Weil das Totarol in den Mikropartikeln gespeichert ist, kann zudem *in vivo* eine große Menge an Totarol vorrätig gehalten werden, ohne dass Totarol seine toxische Wirkung auf die humanen Zellen ausübt. Dies erlaubt die *in vivo* Freisetzung von Totarol über lange Zeiträume.

Bei Verwendung von Resomer 502 ® RG 502 H konnten Mikrokapseln mit einem Gewichtsverhältnis zwischen Totarol und Resomer zwischen 70 und 77% hergestellt werden, deren Durchmesser 60 bis 140 µm betrugen.

Als weitere Inhaltsstoffe können dabei weitere desinfizierende Verbindungen und deren Mischungen vorgesehen sein, um eine möglichst große Breitenwirkung des neuen Mittels gegen vielfältige Mikroorganismen und Krankheitserreger zu erreichen.

Die weiteren desinfizierenden Verbindungen können dabei in eigenen Mikropartikeln gespeichert sein, so dass das erfindungsgemäße Mittel erste, mit Totarol beladene Mikroartikel und zweite, mit zumindest einer anderen Verbindung beladene Mikropartikel enthält.

Dabei ist von Vorteil, dass in dem Mittel zumindest zwei unterschiedliche aktive Substanzen vorhanden sein können, die nicht über längere Zeit in Kontakt miteinander gelagert werden können, jetzt aber in getrennte Partikel geladen sind, so dass sie voneinander getrennt sind.

Vor dem Hintergrund der obigen Entwicklung und der damit gemachten Erfahrungen haben die Erfinder weiter erkannt, dass die in dem erfindungsgemäßen Mittel eingesetzten und mit einer Totarol-Verbindung beladenen Mikropartikel auch in einem nachstehend zur Unterscheidung auch als "Zusammensetzung" bezeichneten Mittel eingesetzt werden können, das als Beschichtung für die Oberflächen von medizinischen Geräten und Implantaten eingesetzt werden kann, vorzugsweise von Gefäßprothesen, weiter vorzugsweise von Außenseiten von Gefäßprothesen, schließlich auch aber nicht abschließend für Thoraco-Abdominal, Iliacal und Popliteal Stents sowie Stentgrafts, periphere Stents, weitere gängige Gefäßprothesen wie z.B. PTFE, Dacron, PU, resorbierbare Polymer- und Saccharidstents, jeweils in dilatierbarer, selbstexpandierbarer, strukturierter oder gecoverter Stentform geeignet sind.

Die Erfindung betrifft daher ein medizinisches Implantat mit einer Oberfläche, auf die eine Beschichtung aufgebracht ist, die Mikropartikel gemäß obiger Beschreibung enthält, die mit einer Totarol-Verbindung beladenen sind.

Die Erfindung betrifft ferner ein medizinisches Implantat, das zumindest teilweise aus resorbierbarem Material, vorzugsweise aus Saccharidverbindungen besteht, wobei das Implantat mit zumindest einem antibakteriell wirkenden Inhaltsstoff beladene Mikropartikel enthält, vorzugsweise die oben beschrieben Mikropartikel enthält, und dass die Mikropartikel in das resorbierbare Material inkorporiert sind, wobei weiter vorzugsweise die neue Zusammensetzung in das resorbierbare Material inkorporiert ist.

Durch das Inkorporieren oder Einmischen der Mikropartikel in die bioresorbierbare Implantatstruktur, beispielsweise in die Stützstruktur einer Gefäßprothese, werden die Inhaltsstoffe aus den Mikropartikeln während der gesamten Abbauzeit des Implantates allmählich abgegeben, entfalten also eine lange Schutzdauer.

Die Mikropartikel können beispielsweise durch Haftvermittler wie hoch viskoses PLGA, PVA oder mit einem biologischen Kleber, beispielsweise Fibrinkleber, auf den Oberflächen der Implantate immobilisiert werden. Versuche der Erfinder zeigen zum Beispiel, dass Gefäßprothesen zuverlässig und stabil mit einer Beschichtung aus mit Totarol beladenen Mikropartikeln versehen werden können, wenn eine 2%-ige Lösung von Polyvinlyalkohol (PVA) als Haftvermittler verwendet wird.

Die Erfindung betrifft ferner ein Beschichtungsmaterial, das zumindest erste mit zumindest einem antibakteriell wirkenden Inhaltsstoff beladene Mikropartikel gemäß obiger Beschreibung enthält.

Dieses Beschichtungsmaterial kann hergestellt und in trockenem Zustand bis zu seiner Verwendung gelagert oder von einem externen Zulieferer bezogen werden.

Erfindungsgemäß umfasst der Inhaltsstoff zumindest eine Totarol-Verbindung, welche vorzugsweise synthetisch hergestelltes oder aus einer natürlichen Quelle gewonnenes Totarol ist.

Die Erfinder haben erstmals erkannt, dass Totarol oder deren Derivate unter anderem zur Behandlung vorliegender pathologischer Gefäßerkrankungen und zur Vermeidung von Protheseninfektionen eingesetzt werden können, insbesondere wenn eingebrachte Gefäßprothesen eine Beschichtung oder eine bioabbaubare Struktur mit Totarol-Gehalt aufweisen. Durch die beladenen Mikropartikel ergibt sich eine prolongierte Freisetzungskinetik über mehrere Wochen, die langwirkend zur Vermeidung von Protheseninfektionen beiträgt.

So berichten Hasse B, Husmann L, Zinkernagel A, Weber R, Lachat M, Mayer D. Vascular graft infections. Swiss Med Wkly. 2013 Jan 24;143, dass Protheseninfektionen schwer zu therapieren sind. Deshalb ist es erfindungsgemäß von großem Vorteil, durch die erfindungsgemäße Beschichtung der Prothesen erst gar keine Adhäsion von Bakterien zugelassen und somit eine konsekutive Biofilmbildung vermieden werden kann.

Keidar Z, Engel A, Hoffman A, Israel O, Nitecki S. J Nucl Med. 2007 Aug;48(8):1230-6. Prosthetic vascular graft infection: the role of 18F-FDG PET/CT, berichten über den relativ späten Zeitpunkt des Auftretens einer Graft Infektion, so dass nicht die Bekämpfung einer Graft-Infektion das Ziel sein darf, sondern die Vermeidung der Entstehung, da einmal generierter Biofilm therapeutisch fast nicht zu behandeln ist und nur noch aggressiv chirurgisch angegangen werden kann.

Bei erfindungsgemäß beschichteten Implantaten wird wegen der verzögerten Freisetzung der Totarol-Verbindung das Auftreten einer Graft-Infektion auch über längere Zeiträume nach der Implantation zuverlässig verhindert.

Wie bereits erwähnt, verfügt Totarol über eine anhaltend hohe Toxizität. Deshalb wäre die erfindungsgemäße Verwendung der Totarol-Verbindung ohne einen zusätzlichen von den Erfindern entwickelten Freisetzungsmechanismus über einen längeren Zeitraum humanmedizinisch nicht möglich. Weil Infektionen auch zeitversetzt zu operativen Eingriffen auftreten, ist eine längerfristige Freisetzung zur Bekämpfung von Bakterienstämmen äußerst vorteilhaft.

Versuche der Erfinder zeigen zudem, dass Totarol und mit Totarol beladene Mikropartikel keine negativen Auswirkungen auf das hämostatische System haben, so dass die erfindungsgemäßen Verwendungen von Totarol oder von mit Totarol beladenen Mikropartikeln keinen Bedenken begegnen.

Als medizinische Implantate kommen dabei vorzugsweise aber nicht abschließend Herzschrittmacher und deren Elektroden, künstliche Hüften, Knochenersatz, Marknägel, Patches, Meshes, selbstexpandierbare und ballonexpandierbare, bioresorbierbare und nicht bioresorbierbare Stents und covered Stents, Endografts, Urostents, Brachialstents etc. in Frage, deren mit Gewebe in Kontakt kommenden Oberflächen erfindungsgemäß beschichtet werden.

Auch können medizinische Geräte, zu denen auch Instrumente und beispielsweise Katheter oder Ballone zählen, mit einer Beschichtung versehen werden, die mit Totarol beladene Mikropartikel enthält, um bei Kontakt mit Gewebe Infektionen zu behandeln oder zu vermeiden.

Dabei kann der Inhaltsstoff bevorzugt zusätzlich eine weitere pharmakologische Verbindung enthalten, vorzugsweise cytostatische bzw. cytotoxische Medikamente wie z.B. Rapamycin, Paclitaxel, weitere Antibiotika, (z.B. Minocyclin-Rifampicin), Silber, Nanosilber, Sulfonamine, antimikrobielle Peptide (AMPs).

Besonders bevorzugt ist es, wenn das Implantat eine Gefäßprothese, ein Stentgraft oder eine Gefäßstütze ist, auf deren jeweilige Außenseite die neue Zusammensetzung als Beschichtung aufgebracht ist.

Gefäßprothesen sind Implantate, die in den Körper eingebracht werden, um geschädigte Abschnitte natürlicher Blutgefäße zu ersetzen. Gefäßprothesen umfassen Kunststoffschläuche, die an die Stümpfe von Blutgefäßen angenäht werden.

Wenn die künstlichen Blutgefäße von innen durch metallische Strukturen abgestützt werden, spricht man von Stentgrafts.

Gefäßstützen im Sinne der vorliegenden Erfindung sind intravaskuläre Implantate, die auch als Stents bezeichnet werden. Derartige Stents sind radial expandierbare Endoprothesen, die transluminal in Gefäße, wie beispielsweise Blutgefäße, Speiseröhre, Luftröhre, Darmkanal etc. implantiert und dann radial expandiert werden, so dass sie sich innen an die Wand des Gefäßes anlegen.

Stents werden bspw. verwendet, um Blutgefäße bei Aneurysmen, Läsionen, Stenosen zu behandeln und zu verstärken und/oder im vaskulären System eine Restenose zu verhindern. Sie können selbstexpandierend sein oder durch eine von innen ausgeübte radiale Kraft aktiv expandiert werden, wenn sie bspw. auf einem Ballon montiert sind.

Unter einer "radial expandierender Gefäßstütze" werden daher im Rahmen der vorliegenden Erfindung sowohl selbstexpandierende als auch aktiv expandierbare Gefäßstützen verstanden.

Die Gefäßstützen weisen dabei einen hohlzylindrischen Körper mit einer Wand aus miteinander verbundenen Streben und Ästen auf, die eine radial durchlässige und federnde Struktur bilden können. Es ist auch bekannt, nicht durchlässige (gecoverte) Stents einzusetzen. Die Stents können selbstexpandierend oder ballonexpandierbar ausgebildet sein.

Der Außendurchmesser des Körpers entspricht im expandierten Zustand etwa dem Innendurchmesser des zu stützenden Gefäßes, an dessen Wandung die Gefäßstütze unter Ausübung einer durch ihre flexible und federnde Struktur bedingten radialen Kraft anliegt. In Längsrichtung ist der Körper der Gefäßstütze für den Durchtritt von in dem gestützten Gefäß transportierten Medien oder Substanzen offen.

Die Gefäßstützen können auch verzweigt ausgebildet sein und/oder seitliche Öffnungen aufweisen, um den Zugang zu abzweigenden Gefäßen zu erhalten bzw. zu ermöglichen.

Insbesondere bei Stents ist es darüber hinaus bekannt, deren Oberflächen luminal oder abluminal mit aktiven Substanzen, insbesondere mit Medikamenten zu beschichten oder Medikamentenreservoirs in der Struktur der Stents vorzusehen bzw. mikroporöse Streben und Äste zu verwenden, um das Medikament zwischenzuspeichern. Das Medikament wird dann lokal an die Gefäßwand abgegeben, um so bspw. Restenosen durch Proliferation des umgebenden Gewebes zu verhindern.

Somit lassen sich durch entsprechend beschichtete oder mit Reservoirs versehene Stents aktive Substanzen gezielt sozusagen vor Ort in das umgebende Gewebe abgeben. Die Beschichtung von Stents, also von Gefäßprothesen, mit aktiven Substanzen ist auch deshalb wünschenswert, weil dadurch die Biokompatibilität der Implantate verbessert wird, wodurch bspw. die Entstehung von Thrombosen bei mit Blut in Kontakt kommenden Oberflächen verhindert werden kann.

Eine derartige Versorgung der Gefäßwand mit Medikamenten ist insbesondere bei Stents mit reiner Metalloberfläche, sogenannten Bare-Metal Stents (BMS) wichtig, denn diese Stents zeigen eine relativ hohe Restenoserate von circa 30%. Grund dafür ist die durch die Implantation ausgelöste Gefäßverletzung, die zu einer Proliferation der glatten Muskelzellen führt.

Um eine Proliferation der glatten Muskelzellen zu verhindern bzw. abzuschwächen, werden Stents daher mit cytostatischen bzw. cytotoxischen Medikamenten wie z.B. Rapamycin oder Paclitaxel beschichtet. Man spricht dann von Drug-Eluting Stents (DES).

Die Medikamente werden dazu mit einem polymeren, biologisch abbaubaren Bindemittel an die Stentoberfläche angebunden oder in eine Polymermatrix auf der Stentoberfläche eingebracht. Nach Implantation der Stents gelangt das Medikament durch Abbau des Polymers in die Gefäßwand.

Als bioresorbierbares Bindemittel werden dabei hauptsächlich verschiedene Polyester auf Basis von Milch- und/oder Glycolsäure verwendet, die sich im Körper ohne Rückstände zersetzen. Bekannt ist die Verwendung von PLA, PLLA und PLGA mit unterschiedlichen Monomerverhältnissen. Diese Polymere unterscheiden sich in ihren mechanischen und chemischen Eigenschaften teilweise deutlich voneinander.

Die WO 2013/ 007589 A1 beschreibt einen Stent, bei dem das Bindemittel ein Oligo(D,L-lactat-co-glycolat) ist, das ein Molekulargewicht (M_{w}) aufweist, das bei etwa 3.000 Dalton liegt, wobei das Monomerenverhältnis von Milchsäure zu Glycolsäure in dem Oligomer etwa 1:1 beträgt.

Das Bindemittel wird als "geschlossene Beschichtung" auf die Außenseite des Stents aufgebracht, die zumindest die gesamte abluminale Oberfläche der Gefäßstütze bedeckt, und auch beim Crimpen und späteren Dilatieren der Gefäßstütze nicht in größeren Bereichen wieder abplatzt. Als aktive Substanz wurde Rapamicin im Gewichtsverhältnis 1:1 mit dem Bindemittel in die geschlossene Beschichtung eingebaut.

Das in der WO 2013/ 007589 A1 als Bindemittel eingesetzte Oligomer ist kommerziell von der Firma Evonik unter dem Handelsnamen Resomer® Condensate 50:50 Mₙ2300 erhältlich. Vergleichsweise wurde ein Polymer, nämlich PLGA mit einem Molekulargewicht von etwa 10.000 Dalton untersucht, das als Resomer® RG 502 H von Evonik vertrieben wird.

In der WO 2013/ 007589 A1 ist gezeigt, dass nur die das Oligomer enthaltende Beschichtung sich bei Gewebekontakt nach 6 Wochen zu mehr als 99% zersetzte.

Aus der EP 1 977 772 A2 ist ein Stent bekannt, auf dessen Oberfläche eine Beschichtung aus PLGA angeordnet ist.

Die Erfinder der vorliegenden Anmeldung haben nun erkannt, dass gerade PLGA zur Herstellung einer Beschichtung aus mit einer Totarol-Verbindung beladenen Mikropartikeln besonders geeignet ist, um eine über mehrere Wochen gleichmäßige Freisetzung des Inhaltsstoffes zu erreichen.

Das war ausgehend von der WO 2013/ 007589 A1 nicht zu erwarten.

Durch die Verwendung von Totarol-Verbindungen als Inhaltsstoff werden bei Verwendung eines Stents oder einer Gefäßprothese mit einer derartigen Beschichtung Protheseninfektionen wirksam verhindert.

Unter "Protheseninfektionen" werden im Rahmen der vorliegenden Erfindung infektiöse Entzündungen verstanden, die pathologischen Ursprung haben oder nach der Implantation auf Oberflächen von Implantaten entstehen, und zwar durch über die Implantatoberfläche oder während der Operation eingeschleppte Mikroorganismen oder bereits im Patienten vorhandene Erreger. Diese Mikroorganismen werden erfindungsgemäß durch die allmähliche Freisetzung des Totarol und/oder der Totarol-Derivate aus den Mikropartikeln über mehrere Wochen vernichtet.

Die neue Zusammensetzung kann dabei mit Totarol-Verbindungen beladene erste Mikropartikel und mit einem weiteren Inhaltsstoff, beispielsweise einer aktiven Substanz wie Rapamicin beladene zweite Mikropartikel enthalten. Dadurch wird die antibakterielle Wirkung von Totarol mit der antiproliferativen Wirkung von cytostatisch bzw. cytotoxisch wirkenden Substanzen kombiniert.

Erfindungsgemäß ist ferner vorgesehen, dass auf der erfindungsgemäßen Beschichtung oder direkt auf der Oberfläche der Implantate eine Außenschicht aufgebracht ist, die eine Totarol-Verbindung, vorzugsweise synthetisch hergestelltes oder aus einer natürlichen Quelle gewonnenes Totarol enthält, wobei weiter vorzugsweise die Außenschicht durch Eintauchen des ggf. mit der Beschichtung versehenen Implantates in eine Lösung hergestellt wird, die eine Totarol-Verbindung, vorzugsweise synthetisch hergestelltes oder aus einer natürlichen Quelle gewonnenes Totarol enthält.

Hier ist von Vorteil, dass die die erfindungsgemäß mit einer Totarol-Verbindung beladenen Mikropartikel enthaltende Beschichtung mit einer Außenschicht aus unverkapseltem Totarol versehen wird, die die Beschichtung versiegelt und nach der Implantation das Totarol schnell in einer gewünschten Menge und in einer ersten Freisetzung (einem sog. Burst) abgibt.

In bestimmten Anwendungsfällen kann es ausreichend sein, auf die Beschichtung mit den Mikropartikeln zu verzichten, und die Totarol-haltige Außenschicht, vorzugsweise durch eine Eintauchbeschichtung, direkt auf der Oberfläche des Implantates anzubringen. Dabei kann durch die Oberflächenstruktur des Implantates auch für eine verzögerte Freisetzung der Totarol-Verbindung gesorgt werden.

Allgemein betrifft die vorliegende Offenbarung daher auch die Verwendung von Totarol-Verbindungen zur Beschichtung von Oberflächen von medizinischen Geräten und Implantaten.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der beigefügten Zeichnung dargestellt und werden in der nachfolgenden Beschreibung näher erläutert. Es zeigen:
- Fig. 1: in schematischer, geschnittener Seitenansicht einen in ein Gefäß eingeführten, mit der erfindungsgemäßen Beschichtung versehenen Stent, vor dessen Dilatation;
- Fig. 2: in schematischer, geschnittener Seitenansicht eine Gefäßprothese mit einer erfindungsgemäßen Beschichtung und einer Außenschicht;
- Fig. 3: ein Balkendiagramm, das erste Meßergebnisse wiedergibt;
- Fig. 4: eine Kurve, die die Freisetzungskinetik für Totarol aus Mikropartikeln über 37 Tage zeigt;
- Fig. 5: den Vergleich zwischen Messungen der optischen Dichte (OD) bei 600 nm für S. aureus Suspensionen, die mit 0,1 mg/ml Totarol (totarol), mit EtAc, oder nicht (untreated) behandelt wurden;
- Fig. 6: Balkendiagramme, die die Wirkung von Totarol und von mit Totarol beladenen Mikropartikeln auf das Hämostatische System zeigen;
- Fig. 7: weitere Balkendiagramme, die die Wirkung von Totarol und von mit Totarol beladenen Mikropartikeln auf das Hämostatische System zeigen;
- Fig. 8: ein Balkendiagramm für die Lebensfähigkeit von HEK-Zellen; und
- Fig. 9: die chemische Formel von Totarol.

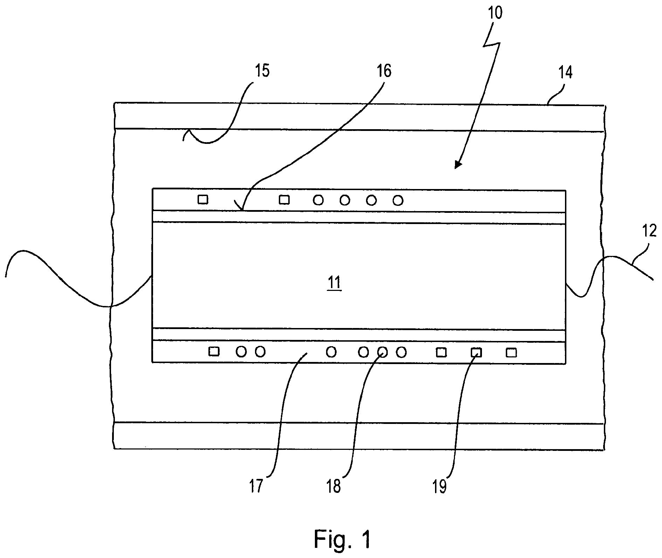

In Fig. 1 ist in schematischer und nicht maßstabsgemäßer Seitenansicht ein Stent 10 gezeigt, der auf einen Ballon 11 aufgecrimpt ist. Der Ballon 11 ist über einen Führungsdraht 12 in ein Gefäß 14 eingeführt worden, mit dessen Wand 15 er nach dem Aufweiten durch den Ballon 11 in Anlage gelangt.

Der Stent 10 weist eine äußere Oberfläche 16 auf, auf die eine bei 17 schematisch angedeutete Beschichtung aufgebracht wurde. Die Beschichtung enthält mit 18 und 19 bezeichnete, erste und zweite Mikropartikel.

Die ersten Mikropartikel 18 wurden auf die nachstehend beschriebene Weise mit Totarol beladen. Die zweiten Mikropartikel 19 wurden mit Rapamycin beladen.

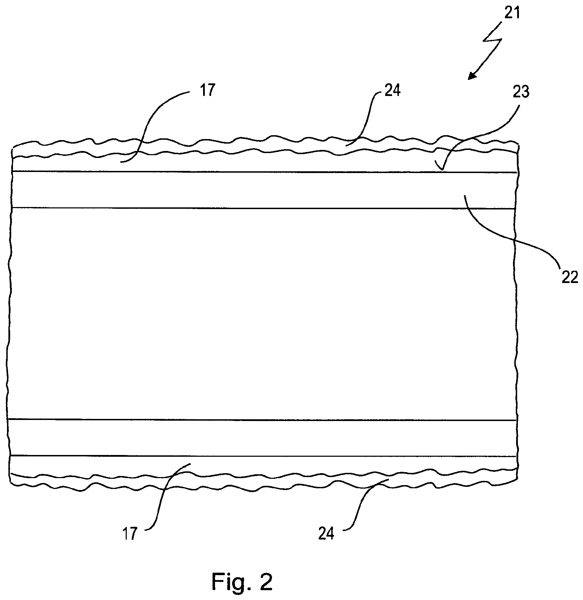

Fig. 2 zeigt in schematischer und nicht maßstabsgemäßer Seitenansicht eine Gefäßprothese 21 mit einer lediglich angedeuteten, hier zylindrischen Wand 22, auf deren Oberfläche 23 die aus Fig. 1 bekannte Beschichtung 17 aufgebracht ist, auf der durch Eintauchbeschichten eine Außenschicht 24 aus einer Totarol-Verbindung aufgebracht ist. In bestimmten Anwendungsfällen kann bei dieser Gefäßprothese 21 auf die Beschichtung 17 auch verzichtet werden, so dass die Außenschicht 24 unmittelbar auf der Oberfläche 23 aufliegt.

Das Eintauchbeschichten wurde an ePTFE Gefäßprothesen mit einer Länge von 3 cm und einem Innendurchmesser von 6 mm getestet. Dazu wurde in einer Petrischale 600 mg Totarol in 5 ml Ethylacetat gelöst und so eine nahezu gesättigte Totarol-Lösung hergestellt. In diese Lösung wurden die Gefäßprothesen für 5 Minuten eingetaucht, so dass sie sich mit Totarol-Lösung vollsaugen konnten, und danach für 30 min getrocknet. Durch Ermittlung der Differenz des Gewichtes der Gefäßprothesen vor und nach dem Tränken und Trockenen konnte die Menge an aufgenommen Totarol zu 30 mg je Gefäßprothese bestimmt werden.

Die mit Totarol beladenen Mikropartikel 18 wurden wie in den nachstehenden Beispielen I und II beschrieben hergestellt.

### Beispiel I: Herstellung W/O/W beladene Totarol Mikropartikel, Evaporationsverfahren

Erster Schritt: Herstellung einer kontinuierlichen (KP) und einer organischen Phase (OP), beide Phasen werden auf einem Magnetrührer hergestellt.

Die KP besteht aus destilliertem. Wasser, Natriumchlorid (NaCl), den Emulgatoren Tween®20 und Polyvinylalkohol (PVA) sowie Natriumhydroxid (NaOH).

Zunächst wird das NaCl in dest. Wasser unter Rühren gelöst (300rpm). Anschließend wird das PVA dazugegeben und unter weiterem Rühren (450 rpm) auf 80°C erhitzt. Diese Temperatur wird so lange gehalten, bis sich das PVA vollständig gelöst hat. Danach kühlt die KP unter weiterem Rühren (600 rpm) auf 40°C ab. Ist diese Temperatur erreicht wird das Tween®20 hinzugegeben und unter weiterem Rühren (500rpm) auf Raumtemperatur abgekühlt

Die parallel zur KP hergestellte OP besteht aus dem Wirkstoff Totarol, dem Polymer Resomer® 502, Ethylacetat und dem Tensid Brij®30.

Zunächst wird der Wirkstoff Totarol und das Resomer®502 vorgelegt und unter Rühren (230 rpm) in Ethylacetat gelöst. Nach ca. 5 min sollten alle Stoffe vollständig gelöst sein. Unter weiterem Rühren (230 rpm) wird Brij®30 dazugegeben und weitere 5 min gerührt.

Anschließend werden in einem ersten Schritt die beiden Phasen in einem Verhältnis von 1:1 ineinander emulgiert. Hierfür wird die KP tropfenweise (1ml pro Minute) zu der OP gegeben. Dieser Schritt findet in einem Becherglas unter ständigem Rühren (280 rpm) statt. Hierbei wird die Präemulsion (W/O) gebildet, welche für 20 min bei derselben Drehzahl gerührt wird.

Im nächsten Schritt wird erneut ein Teil der KP zur Präemulsion gegeben, genau das 7-fache des eingesetzten Lösungsmittels in der OP. Dadurch wird die multiple Emulsion W/O/W gebildet. Nach der Zugabe wird weiter 1 h bei Raumtemperatur gerührt (300 rpm). Die Mikropartikel beginnen in dieser Zeit auszuhärten.

Im letzten Schritt wird noch einmal ein Teil der KP hinzugegeben, und zwar das 19-fache des eingesetzten Lösungsmittels in der OP. Es wird weiter 1 h gerührt (300 rpm), wobei die Temperatur auf 24°C erhöht wird.

In den darauffolgenden Schritten werden die Mikropartikel abgesiebt und drei mal mit dest. Wasser gewaschen. Anschließend werden sie für 24 Stunden luftgetrocknet.

### Beispiel II: Herstellung O/W beladene Mikropartikel, Evaporationsverfahren

Erster Schritt: Herstellung einer kontinuierlichen- (KP) und einer organischen Phase (OP), beide Phasen werden auf einem Magnetrührer hergestellt.

Die KP besteht aus destilliertem Wasser, Natriumchlorid (NaCl), den Emulgatoren Tween®20 und Polyvinylalkohol (PVA)

Zunächst wird das NaCl in dest. Wasser unter Rühren gelöst (300rpm). Anschließend wird das PVA dazugegeben und unter weiterem Rühren (450 rpm) auf 80°C erhitzt. Diese Temperatur wird so lange gehalten bis sich das PVA vollständig gelöst hat. Danach kühlt die KP unter weiterem Rühren (600 rpm) auf 40°C ab. Ist diese Temperatur erreicht wird das Tween®20 hinzugegeben und unter weiterem Rühren (500rpm) auf Raumtemperatur abgekühlt OP: Diese Phase wird parallel zur KP hergestellt.

Die OP besteht aus dem Wirkstoff Totarol, dem Polymer Resomer® 502, Ethylacetat und dem Tensid Brij®30.

Zunächst wird der Wirkstoff Totarol und das Resomer®502 vorgelegt und unter Rühren (230 rpm) in Ethylacetat gelöst. Nach ca. 5 min sollten alle Stoffe vollständig gelöst sein. Unter weiterem Rühren (230 rpm) wird Brij®30 dazugegeben und weitere 5 min gerührt.

Das 40-fache des eingesetzten Lösungsmittels in der OP wird als KP vorgelegt und die OP wird ohne zu Rühren komplett in die KP gegeben. Anschließend wird für 1,5 min bei 1100 rpm gerührt. Nach dieser Zeit wird die Drehzahl auf 350 rpm reduziert und für 3,5 h bei 25°C gerührt.

In den darauffolgenden Schritten werden die Mikropartikel abgesiebt und dreimal mit dest. Wasser gewaschen. Anschließend werden sie für 24 Stunden luftgetrocknet.

Der Gewichtsanteil des Totarol an den Mikropartikeln betrug je nach Charge zwischen 70 und 77%, die Durchmesser der Mikropartikel lagen bei 60 bis 140 µm.

Die mit Totarol beladenen Mikropartikel wurden dann auf ihre antibakterielle Wirkung gegen Streptococcus gordonii (S.G.) getestet. Dazu wurden 5 mg von Totarol beladenen Mikropartikel in 6-Well-Platten gegeben mit einer Lösung von S.G. überschichtet.

Als Vergleich wurden 5 mg unbeladene Mikropartikel, 5 mg freies Totarol, reines Medium (Schaedler Medium der Firma BD), Medium mit S.G. und Medium mit S.G. und 4% P/S (Penicillin Streptomycin) als Positivkontrolle gemessen.

Anhand der Veränderung der optischen Dichte wurde das Wachstum von S.G. bestimmt. Die Startdichte für S.G. war 1,8.

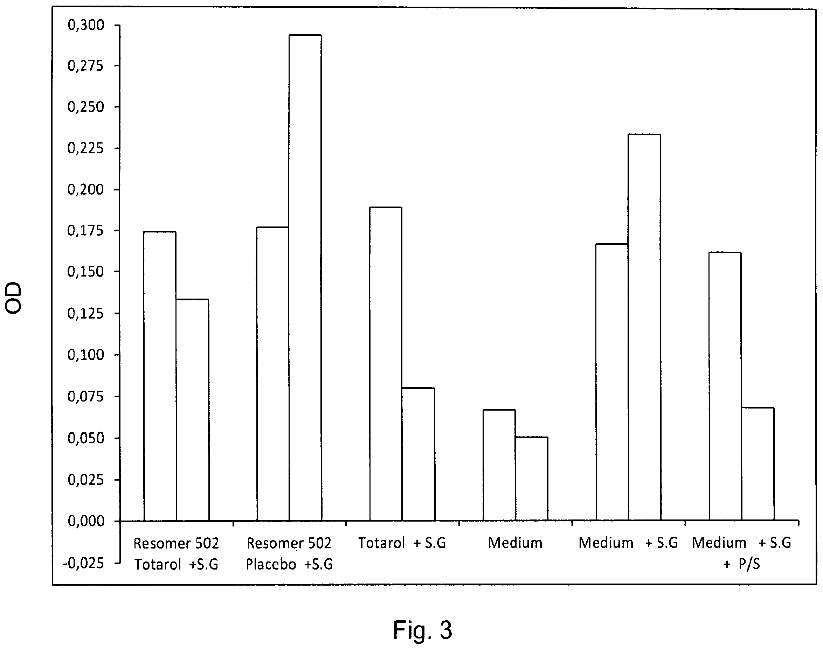

In Fig. 3 sind die Messergebnisse zur Stunde 0 (linke Balken) und nach 24 Stunden (rechte Balken) dargestellt.

Es ist zu erkennen, dass S.G. in den Ansätzen mit unbeladenen Mikropartikeln und reinem Medium gewachsen sind, während freies Totarol und P/S zu einem stärkeren Rückgang der Bakterienkonzentration führte als mit Totarol beladenene Mikropartikel. Dies zeigt, dass das verkapselte Totarol aktiv ist, aber langsamer wirkt als freies Totarol.

### Beispiel III: antibakterielle Wirkung von mit Totarol beladenen Mikropartikeln

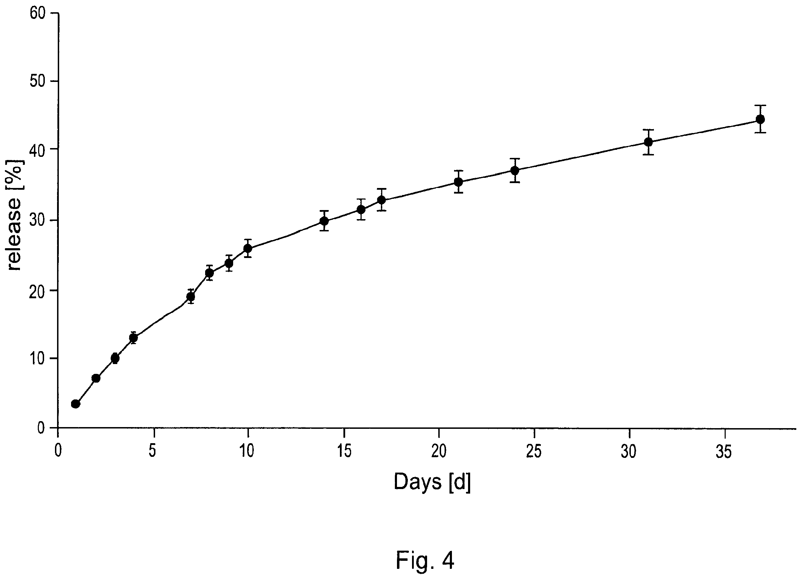

Die mittlere Partikelgröße der nach den oben beschriebenen Verfahren hergestellten, mit Totarol beladenen Mikropartikel betrug in einem Versuch 156 um. Die Menge an verkapseltem Totarol betrug 90% des eingesetzten Totarols. Die Freisetzungskinetik des Totarol ist in Fig. 4 dargestellt. Danach erfolgte die Freisetzung kontinuierlich über mindestens 37 Tage zu 50%.

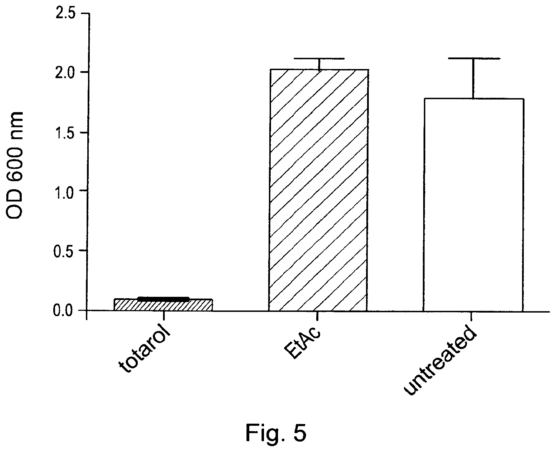

Die antibakterielle Wirkung von Totarol und von mit Totarol beladenen Mikropartikeln auf Staphylococcus aureus (S. aureus) wurde in einem weiteren Versuch *in vitro* getestet. Es zeigte sich, dass eine Konzentration von 0,1 mg/ml an in Ethylacetat (EtAc) gelöstem Totarol ausreichend ist, um das Wachstum von S. aureus in Suspension nach 6 Stunden Inkubation zu inhibieren. Fig. 5 zeigt den Vergleich zwischen Messungen der optischen Dichte (OD) bei 600 nm für S. aureus Suspensionen, die mit 0,1 mg/ml Totarol (totarol), mit EtAc, oder nicht (untreated) behandelt wurden.

In weiteren Versuchen wurden die S. aureus Bakterien auf Agarplatten ausgesät und 1 mg an reinem Totarol, 10 mg an unbeladenen Mikropartikeln, und 10 mg an mit ungefähr 1 mg an Totarol beladenen Mikropartikeln behandelt. Nach 24 Stunden Inkubation bei 37°C zeigten sowohl die Kultur mit reinem Totarol als auch die Kultur mit den beladenen Mikropartikeln eine klare Inhibitionszone.

Der Versuch wurde mit Filterpapier wiederholt, das mit reinem Totarol, mit mit Totarol beladenen Mikropartikeln und mit EtAc getränkt war und auf Agarplatten inkubiert wurde, auf denen S. aureus kultiviert wurden. Nach 24 Stunden Inkubation bei 37°C zeigten verglichen mit der EtAc-Kontrolle sowohl die Kultur mit reinem Totarol als auch die Kultur mit den beladenen Mikropartikeln um das Filterpapier eine klare Inhibitionszone.

### Beispiel IV: Hämokompatibilität von mit Totarol beladenen Mikropartikeln

Schließlich wurden *in vitro* Tests durchgeführt, in denen Totarol und mit Totarol beladenen Mikropartikeln mit Vollblut in Kontakt gebracht wurde. Untersucht wurden die Wirkung auf Erythrozyten, Plättchen, Leukozyten sowie die Komplement- und Koagulationskaskade. Die Hämokompatibilität wurde mit Konzentrationen an Totarol und an mit Totarol beladenen Mikropartikeln getestet, die *in vitro* eine hinreichende antibakterielle Wirkung zeigen.

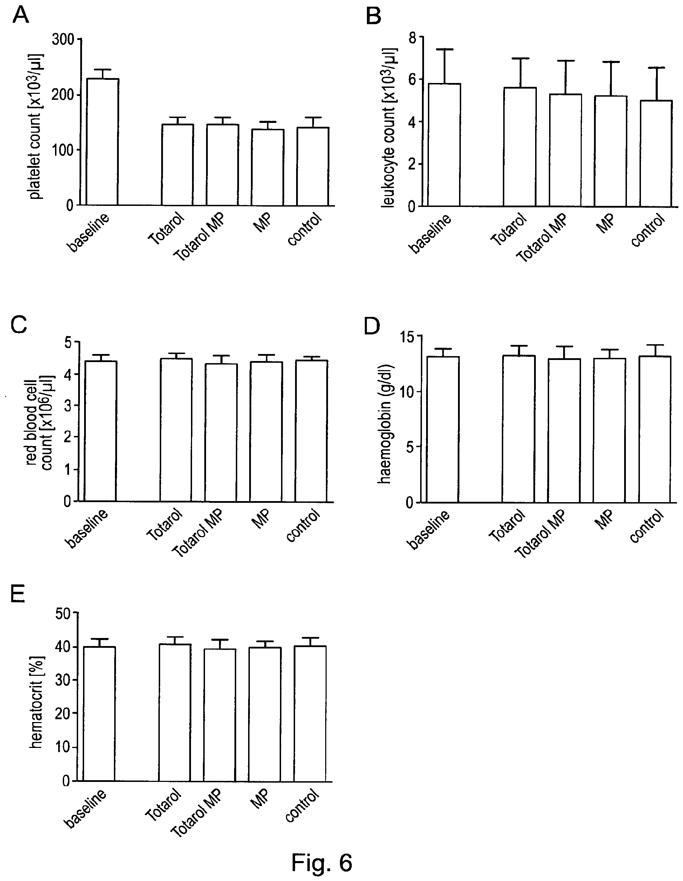

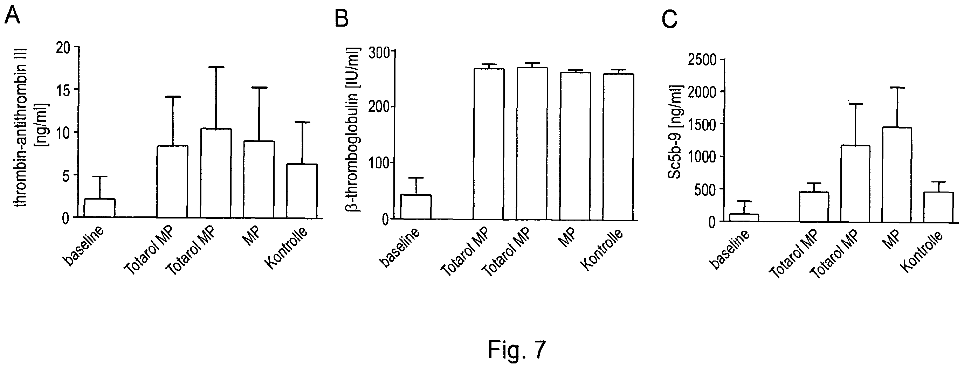

Die Messungen wurden gemäß ISO 10993-4 durchgeführt, indem verschiedene Marker mit zentraler Bedeutung innerhalb des Hämostatischen Systems bestimmt wurden. Die Daten zeigen, dass verglichen mit der Kontrollgruppe und der Behandlung mit unbeladenen Mikropartikeln weder Totarol noch die mit Totarol beladenen Mikropartikel negative Wirkungen auf das Hämostatische System haben; siehe Fig. 6 und Fig. 7

Fig. 6 zeigt Balkendiagramme für Plättchen-, Leukozyten- und Erythrozyten-Anzahl sowie für Hämoglobin- und Hämatokrit-Werte, jeweils gemessen vor (baseline) und nach der Behandlung von frischem humanem Vollblut mit 0,1 mg/ml Totarol (Totarol), mit 1 mg/ml an mit Totarol beladenen Mikropartikeln (Totarol MP), mit 1 mg/ml unbeladenen Mikropartikeln (MP) und Puffer (control), jeweils inkubiert für 1 Stunde. Gezeigt sind Mittelwerte und Standardabweichungen (n=3).

Fig. 7 zeigt Balkendiagramme für Thrombin-Antithrombin III-, Beta-Thromboglobulin- und Sc5b-9-Konzentrationen, jeweils gemessen vor (baseline) und nach der Behandlung von frischem humanem Vollblut mit 0,1 mg/ml Totarol (Totarol), mit 1 mg/ml an mit Totarol beladenen Mikropartikeln (Totarol MP), mit 1 mg/ml unbeladenen Mikropartikeln (MP) und Puffer (control), jeweils inkubiert für 1 Stunde. Gezeigt sind Mittelwerte und Standardabweichungen (n=3).

Die Daten zeigen eine gute Hämokompatibilität sowohl für Totarol als auch für die mit Totarol beladenen Mikropartikel. Es konnte keine biologisch relevante Veränderung während des Blutkontaktes beobachtet werden.

### Beispiel V: Wirkung von mit Totarol beladenen Mikropartikeln auf HEK

Die Zellviabilität von humanen embryonalen Nierenzellen (HEK) wurde nach Inkubation mit mit Totarol beladenen Mikropartikeln (Endkonzentration 1 mg/ml) über 24 Stunden mittels MTT analysiert. MTT ist ein Test auf Zellviabilität mittels des Farbstoffes Tetrazolium MTT 3-(4,5-dimethylthiazol-2-yl)-2,5-diphenyltetrazolium bromide.

Die Ergebnisse zeigen, dass mit Totarol beladene Mikropartikel keinen Einfluss auf die Lebensfähigkeit haben, wenn sie in Konzentrationen eingesetzt werden, die antimikrobiell wirksam sind.

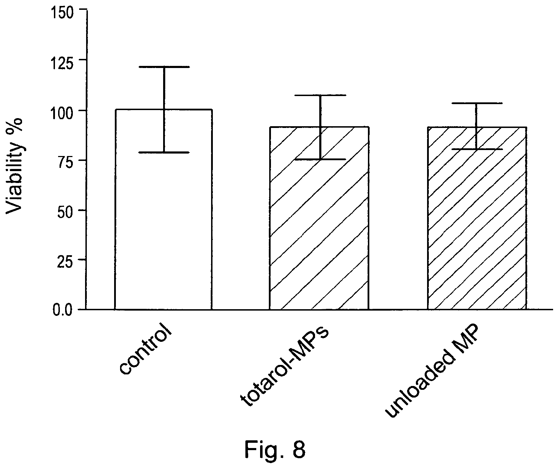

Fig. 8 zeigt ein Balkendiagramm für die Lebensfähigkeit von HEK-Zellen nach 24 Stunden Inkubation mit 1 mg/ml an mit Totarol beladenen Mikropartikeln (totarol MPs) und mit 1 mg/ml an unbeladenen Mikropartikeln (unloaded MPs) im Vergleich zu einer unbehandelten Kontrolle (control). Gezeigt sind Mittelwerte und Standardabweichungen (n=3).

## Patentansprüche

1. Mittel zur reinigenden, desinfizierenden und/oder schützenden Behandlung von technischen Oberflächen, mit zumindest einem antibakteriell wirkenden Inhaltsstoff, wobei es mit dem antibakteriell wirkenden Inhaltsstoff beladene Mikropartikel enthält, **dadurch gekennzeichnet, dass** die Mikropartikel aus einem Poly (Lactid-co-glycolid) mit einem Lactid-Glycolid-Verhältnis von 50:50 und einem Molekulargewicht kleiner als 30.000 g/Mol hergestellt sind, und dass der Inhaltsstoff zumindest Totarol umfasst.

2. Mittel nach Anspruch 1, **dadurch gekennzeichnet, dass** die Mikropartikel biodegradierbare Mikrokapseln oder Mikrosphärulen sind.

3. Mittel nach einem der Ansprüche 1 bis 2, **dadurch gekennzeichnet, dass** das Mittel ein Lösungsmittel für die mit dem Inhaltsstoff beladenen Mikropartikel enthält.

4. Mittel nach Anspruch 3, **dadurch gekennzeichnet, dass** das Lösungsmittel ausgewählt ist, aus der Gruppe, die Hexan, Aceton, Ethanol, Acetonitril, Chloroform, DSMO (Dimethyl Sulfoxid), Methanol, Ethylacetat und das Tensid Polyoxyethylen(4)laurylether umfasst.

5. Mittel nach einem der Ansprüche 1 bis 4, **dadurch gekennzeichnet, dass** das Mittel als Spray, in einem getränkten Tuch, insbesondere Reinigungstuch, Wundauflage, Abdecktuch, einer getränkten Mundschutzmaske, getränkter Operationskleidung, oder flüssig vorliegt.

6. Mittel nach einem der Ansprüche 1 bis 5, **dadurch gekennzeichnet, dass** der Inhaltstoff synthetisch hergestelltes oder aus natürlichen Quellen gewonnenes Totarol ist.

7. Mittel nach einem der Ansprüche 1 bis 6, **dadurch gekennzeichnet, dass** der Inhaltstoff zusätzlich eine weitere desinfizierende Verbindung enthält.

8. Zusammensetzung, die das Mittel nach einem der Ansprüche 1 bis 7 umfasst.

9. Beschichtungsmaterial, das die Zusammensetzung nach Anspruch 8 enthält.

10. Medizinisches Implantat mit einer Oberfläche, auf die eine Beschichtung aufgebracht ist, die das Beschichtungsmaterial nach Anspruch 9 enthält.

11. Medizinisches Implantat nach Anspruch 10, **dadurch gekennzeichnet, dass** es eine Gefäßprothese, eine Gefäßstütze oder ein Stentgraft ist.

12. Medizinisches Implantat nach Anspruch 11, **dadurch gekennzeichnet, dass** die Oberfläche eine Außenseite der Gefäßprothese ist.

13. Verwendung des Mittels nach einem der Ansprüche 1 bis 7 zur Reinigung, zur Desinfektion, zur Dekontamination und/oder zum Schutz von technischen Oberflächen.

## Claims

1. Means for a cleaning, disinfecting and/or protecting treatment of technical surfaces, comprising at least one antibacterially effective ingredient, wherein it contains microparticles loaded with the antibacterially effective ingredient, **characterized in that** the microparticles are made of a poly(lactide-co-glycolide) with a lactide-glycolide ratio of 50:50 and a molecular weight of smaller than 30.000 g/mole, and that the ingredient comprises at least totarol.

2. Means of claim 1, **characterized in that** the microparticles are biodegradable microcapsules or microspherules.

3. Means of any of claims 1 to 2, **characterized in that** the means contains a solvent for the microparticles loaded with the ingredient.

4. Means of claim 3, **characterized in that** the solvent is selected from the group consisting of hexane, acetone, ethanol, acetonitrile, chloroform, DSMO (dimethyl sulfoxide), methanol, ethyl acetate and the tenside polyoxyethylene(4)lauryl ether.

5. Means of any of claims 1 to 4, **characterized in that** the means is provided as a spray, in a soaked cloth, in particular in a cleaning cloth, a wound dressing, a cover cloth, a soaked mouth mask, a soaked surgical clothing, or in liquid form.

6. Means of any of claims 1 to 5, **characterized in that** the ingredient is synthetically produced totarol or totarol from a natural source.

7. Means of any of claims 1 to 6, **characterized in that** the ingredient additionally contains a further disinfecting compound.

8. Composition which comprises the means of any of claims 1 to 7.

9. Coating material comprising the composition of claim 8.

10. Medical implant with a surface on which a coating is provided which comprises the coating material of claim 9.

11. Medical implant of claim 10, **characterized in that** it is a vascular prosthesis, a vascular support or a stent graft.

12. Medical implant of claim 11, **characterized in that** the surface is an outer surface of the vascular prosthesis.

13. Use of a means of any of claims 1 to 7 for the cleaning, for the disinfection, for the decontamination and/or for the protection of technical surfaces.

## Revendications

1. Agent permettant le traitement nettoyant, désinfectant et/ou protecteur de surfaces techniques, avec au moins un constituant à effet antibactérien, dans lequel il contient des microparticules chargées du constituant à effet bactérien, **caractérisé en ce que** les microparticules sont fabriquées à partir d'un poly(lactide-co-glycolide) avec un rapport lactide-glycolide de 50:50 et un poids moléculaire inférieur à 30 000 g/Mol, et que le constituant comprend au moins du totarol.

2. Agent selon la revendication 1, **caractérisé en ce que** les microparticules sont des microcapsules ou microsphérules biodégradables.

3. Agent selon l'une quelconque des revendications 1 et 2, **caractérisé en ce que** l'agent contient un solvant pour les microparticules chargées du constituant.

4. Agent selon la revendication 3, **caractérisé en ce que** le solvant est choisi dans le groupe qui comprend l'hexane, l'acétone, l'éthanol, l'acétonitrile, le chloroforme, le DSMO (diméthylsulfoxyde), le méthanol, l'acétate d'éthyle et le tensioactif polyoxyéthylène(4)lauryléther.

5. Agent selon l'une quelconque des revendications 1 à 4, **caractérisé en ce que** l'agent se présente en tant que spray, dans une toile imprégnée, en particulier une toile de nettoyage, un pansement, champ, un masque chirurgical imprégné, vêtement opératoire imprégné, ou sous forme liquide.

6. Agent selon l'une quelconque des revendications 1 à 5, **caractérisé en ce que** le constituant est du totarol fabriqué de manière synthétique ou extrait de sources naturelles.

7. Agent selon l'une quelconque des revendications 1 à 6, **caractérisé en ce que** le constituant contient en plus un autre composé désinfectant.

8. Composition qui comprend l'agent selon l'une quelconque des revendications 1 à 7.

9. Matériau de revêtement qui contient la composition selon la revendication 8.

10. Implant médical avec une surface sur laquelle un revêtement est appliqué, qui contient le matériau de revêtement selon la revendication 9.

11. Implant médical selon la revendication 10, **caractérisé en ce qu'**il est une prothèse vasculaire, une endoprothèse ou une endoprothèse couverte.

12. Implant médical selon la revendication 11, **caractérisé en ce que** la surface est une face extérieure de la prothèse vasculaire.

13. Utilisation de l'agent selon l'une quelconque des revendications 1 à 7 pour le nettoyage, pour la désinfection, pour la décontamination et/ou pour la protection de surfaces techniques.
